# EUROPÄISCHE PATENTSCHRIFT

(11) **EP 1 444 205 B1**
(45) Veröffentlichungstag und Bekanntmachung des Hinweises auf die Patenterteilung: **01.02.2006**
(21) Anmeldenummer: 02783066.0
(22) Anmeldetag: 31.10.2002
(51) Int. Cl.: C07D 213/81, C07D 213/82

(54) **VERFAHREN ZUR HERSTELLUNG VON 2-HALOGENPYRIDINCARBONSÄUREAMIDEN**
METHOD FOR PRODUCING 2-HALOGEN-PYRIDINE-CARBOXYLIC ACID AMIDES
PROCEDE POUR LA PRODUCTION D'AMIDES D'ACIDE 2-HALOGENE-PYRIDINE-CARBOXYLIQUE

(30) Priorität: 02.11.2001 EP 01126113
(43) Veröffentlichungstag der Anmeldung: 11.08.2004
(73) Patentinhaber: BASF Aktiengesellschaft, 67056 Ludwigshafen (DE)
(72) Erfinder: MAYER, Horst, 67069 Ludwigshafen (DE); GOLSCH, Dieter, 68165 Mannheim (DE); ISAK, Heinz, 67459 Böhl-Iggelheim (DE); SCHRÖDER, Jochen, 67245 Lambsheim (DE)
(74) Vertreter: Reitstötter - Kinzebach
(86) Internationale Anmeldenummer: PCT/EP2002/012214
(87) Internationale Veröffentlichungsnummer: WO 2003/037868

(56) Entgegenhaltungen:
- EP-A- 0 545 099
- US-A- 2 186 773
- AUGERI, DAVID J. ET AL: "Potent and Selective Non-Cysteine-Containing Inhibitors of Protein Farnesyltransferase" J. MED. CHEM. (1998), 41(22), 4288-4300, XP002189278

## Beschreibung

Die vorliegende Erfindung betrifft ein Verfahren zur Herstellung von 2-Halogenpyridincarbonsäureamiden, insbesondere von 2-Halogennicotinsäureamiden primärer aromatischer Monoamine, die in der ortho-Position zur Aminogruppen einen von Wasserstoff verschiedenen Substituenten aufweisen.

2-Halogenpyridincarbonsäureamide von primären aromatischen Monoaminen I, welche in der ortho-Position zur Aminogruppe einen von Wasserstoff verschiedenen Substituenten aufweisen, sind als fungizide Wirkstoffe aus der EP-A 545 099 bekannt. Ihre Herstellung erfolgt durch Umsetzung von 2-Halogenpyridincarbonsäurechloriden mit ortho-substituierten aromatischen Monoaminen in Gegenwart einer Base, vorzugsweise einem tertiären Amin, in einem organischen Lösungsmittel. Die Base dient zur Bindung des bei der Reaktion entstehenden Halogenwasserstoffs und wird daher in einer wenigstens stöchiometrischen Menge eingesetzt. Nachteilig hierbei ist, daß geringe Basenmengen im primär anfallenden Reaktionsprodukt verbleiben und durch aufwendige Reinigungsmaßnahmen entfernt werden müssen, um spezifikationsgerechten Wirkstoff herzustellen. Davon abgesehen, ist der Einsatz einer Base ein zusätzlicher Kostenfaktor bei der Herstellung dieser Wirkstoffe.

Grundsätzlich besteht daher Interesse an einem Verfahren zur Herstellung dieser Wirkstoffe, das ohne den Einsatz einer Base auskommt. Dabei ist jedoch zu berücksichtigen, daß ortho-substituierte aromatische Monoamine, im folgenden Monoamine I, aufgrund des ortho-Substituenten sterisch gehindert und damit vergleichsweise reaktionsträge sind. Hinzu kommt, daß die aromatischen Monoamine I hinreichend basisch sind, um von dem bei der Reaktion entstehenden Halogenwasserstoff protoniert zu werden, so dass bei Abwesenheit einer Base in der Regel nur Teilumsätze erreicht werden. Der Einsatz stöchiometrischer Mengen einer Hilfsbase bei der Umsetzung von 2-Halogenpyridincarbonsäurechloriden II mit aromatischen, ortho-substituierten Monoaminen I wurde daher bislang als erforderlich erachtet.

Der vorliegenden Erfindung liegt die Aufgabe zu Grunde, ein Verfahren zur Herstellung von 2-Halogenpyridincarbonsäureamiden durch Umsetzung primärer aromatischer Monoamine I mit 2-Halogenpyridincarbonsäurechloriden II bereit zu stellen, welches die gewünschten 2-Halogenpyridincarbonsäureamide in hoher Ausbeute liefert, ohne daß ein äquimolarer Einsatz von Base erforderlich ist. Das Verfahren soll insbesondere für die Umsetzung von 2-Halogenpyridincarbonsäurechloriden mit den besonders reaktionsträgen aromatischen Monoaminen von 2-Aminobiphenyl-Typ geeignet sein. Zudem soll das Verfahren auch im großen Maßstab durchführbar sein.

Diese Aufgabe konnte überraschenderweise gelöst werden durch ein Verfahren, bei dem man 2-Halogenpyridincarbonsäurechlorid II mit einem aromatischen Monoamin I in einem Lösungsmittelgemisch, umfassend Wasser und wenigstens ein mit Wasser nicht mischbares organisches Lösungsmittel, in weitgehender oder vollständiger Abwesenheit einer Hilfsbase umsetzt. Die bei dieser Umsetzung erreichten hohen Ausbeuten sind insbesondere auch deswegen überraschend, da Pyridincarbonsäurechloride äußerst hydrolyselabil sind. In der Regel werden daher Pyridincarbonsäurechloride nur unter wasserfreien Bedingungen umgesetzt.

Demnach betrifft die vorliegende Erfindung ein Verfahren zur Herstellung von 2-Halogenpyridincarbonsäureamiden primärer aromatischer Monoamine I, die in der ortho-Position zur Aminogruppe einen von Wasserstoff verschiedenen Substituenten aufweisen, durch Umsetzung von 2-Halogenpyridincarbonsäurechlorid II mit dem aromatischen Monoamin I, dadurch gekennzeichnet, dass man die Umsetzung in einer Lösungsmittelmischung aus Wasser und wenigstens einem unter den salzsauren Reaktionsbedingungen mit Wasser nicht mischbaren organischen Lösungsmittel durchführt, das ausgewählt ist unter aromatischen Kohlenwasserstoffen, Halogenkohlenwasserstoffen, acyclischen Ethern mit 4 bis 10 C-Atomen, 3 bis 10 C-Atome aufweisenden Estern aliphatischer oder cycloaliphatischer Alkohole mit aliphatischen Carbonsäuren und Mischungen dieser Lösungsmittel, wobei die Mischung unter den salzsauren Reaktionsbedingungen wenigstens eine organische und wenigstens eine wässrige Phase umfasst, und wobei die Mischung keine oder weniger als 10 mol-%, bezogen auf das 2-Halogenpyridincarbonsäurechlorid II, einer von I und II verschiedenen Base enthält, und die Gesamtmenge von Verbindung I und II 25 Gew.-Teile bis 100 Gew.-Teile, bezogen auf 100 Gew.-Teile organisches Lösungsmittel, beträgt.

Unter mit Wasser nicht mischbaren organischen Lösungsmitteln versteht man hierin organische Lösungsmittel und Lösungsmittelmischungen, die unter den salzsauren Reaktionsbedingungen beim Mischen mit Wasser ein wenigstens eine organische und wenigstens eine wässrige Phase umfassendes Mehrphasensystem ausbilden. In der Regel handelt es sich dabei um solche Lösungsmittel, in denen sich weniger als 10 Vol.-% Wasser bzw. verdünnte Salzsäure lösen. Geeignet sind auch Lösungsmittelgemische, die neben den vorgenannten Lösungsmitteln mit Wasser mischbare, aprotische Lösungsmittel enthalten, da auch diese Gemische unter den Reaktionsbe-dingungen mit Wasser ebenfalls ein Zweiphasensystem ausbilden können.

Zu den mit Wasser nicht mischbaren organischen Lösungsmitteln zählen beispielsweise aromatische Kohlenwasserstoffe, aromatische, aliphatische und cycloaliphatische Halogenkohlenwasserstoffe, acyclische Ether mit 4 bis 10 C-Atomen, 3 bis 10 C-Atome aufweisende Ester aliphatischer oder cycloaliphatischer Alkohole mit aliphatischen Carbonsäuren, z.B. Ester der Essigsäure, der Propionsäure oder der Buttersäure mit C₃-C₈-Alkanolen wie Methyl-, Ethyl-, n-Propyl-, n-Butyl- oder iso-Butylacetat, -propionat, -butyrat etc. sowie Mischungen der vorgenannten organischen Lösungsmittel. Als mit Wasser mischbare Lösungsmittel kommen z.B. Aceton, cyclische Ether wie Tetrahydrofuran, Dioxan, weiterhin Acetonitril oder Propionitril in Betracht. Der Anteil an mit Wasser mischbaren Lösungsmitteln wird in der Regel nicht mehr als 50 Gew.-%, vorzugsweise nicht mehr als 20 Gew.-%, bezogen auf die Gesamtmenge an organischem Lösungsmittel betragen. In einer bevorzugten Ausführungsform ist das eingesetzte Lösungsmittel im wesentlichen frei von mit Wasser mischbaren Lösungsmitteln (Anteil < 5 Gew.-%).

Eingesetzt werden solche organischen Lösungsmittel, in denen sich zumindest die Edukte I und II hinreichend lösen. Bevorzugt sind insbesondere solche Lösungsmittel, in denen sich Eduktkonzentrationen von wenigstens 20 Gew.-% und insbesondere wenigstens 30 Gew.-% erreichen lassen. Beispiele für bevorzugte organische Lösungsmittel sind aromatische, vorzugsweise Alkyl-substituierte Kohlenwasserstoffe wie Toluol, Ethylbenzol, o-, m- und p-Xylol, Cumol und p-Methylcumol, Halogenkohlenwasserstoffe, insbesondere Chlorkohlenwasserstoffe wie Dichlormethan, Trichlormethan, 1,2-Dichlorethan, Chlorbenzol und Dichlorbenzole, Ether, z.B. C₂-C₄-Dialkylether wie Diethylether, Diisopropylether, Di-n-butylether, Di-sec.-butylether sowie Methyl-tert.-butylether, weiterhin sowie die vorgenannten Ester mit 3 bis 10 C-Atomen. Geeignet sind selbstverständlich auch Mischungen der vorgenannten Lösungsmittel. Besonders bevorzugte Lösungsmittel und Lösungsmittelgemische enthalten zum überwiegenden Anteil, vorzugsweise zu wenigstens 80 Vol.-% und insbesondere zu wenigstens 90 Vol.-% wenigstens einen aromatischen Kohlenwasserstoff. Als aromatische Kohlenwasserstoffe sind insbesondere C₁-C₄-monoalkyl- und C₁-C₄-Dialkylbenzole, speziell Xylole bevorzugt.

Erfindungsgemäß wird das Verhältnis von Wasser zu organischen Lösungsmitteln so gewählt, daß sich unter Reaktionsbedingungen eine erste Phase, welche das Lösungsmittel und das Reaktionsprodukt enthält, sowie eine zweite wässrige Phase bilden. Die Menge an Wasser wird dabei vorzugsweise so gewählt, daß die wassermenge wenigstens 100 g, vorzugsweise wenigstens 200 g und insbesondere wenigstens 300 g je Mol Pyridincarbonsäurechlorid II beträgt. In der Regel wird man nicht mehr als 1 kg Wasser je Mol 2-Halogenpyridincarbonsäurechlorid II einsetzen.

Die Menge an mit Wasser mischbarem organischen Lösungsmittel wird in der Regel so bemessen, daß das Volumenverhältnis von Wasser zu Lösungsmittel im Bereich von 10:1 bis 1:10 liegt. Nicht zuletzt aus Kostengründen ist es von Vorteil, die eingesetzte Lösungsmittelmenge möglichst gering zu halten. Erfindungsgemäß setzt man daher soviel Lösungsmittel ein, daß die Gesamtmenge der Edukte I und II wenigstens 25 Gew.-Teile, vorzugsweise wenigstens 30 Gew.-Teile und insbesondere wenigstens 50 Gew.-Teile bezogen auf 100 Gew.-Teile des organischen, mit Wasser nicht mischbaren Lösungsmittels beträgt. Vorzugsweise wird man die Lösungsmittelmenge so wählen, daß die Edukte und auch das Pyridincarbonsäureamid unter den Reaktionsbedingugen in dem organischen Lösungsmittel mehr oder weniger vollständig löslich sind, so daß sich eine weitgehend homogene organische Phase ausbilden kann. In der Regel wird man daher wenigstens 100 Gew.-Teile Lösungsmittel und vorzugsweise wenigstens 130 Gew.-Teile Lösungsmittel je 100 Gew.-Teile Edukt (Gesamtmenge von I und II) einsetzen.

Zur Umsetzung des 2-Halogenpyridincarbonsäurechlorids II mit dem aromatischen Monoamin I geht man in der Regel so vor, daß man die Edukte I und II als Lösungen in dem organischen Lösungsmittel in Gegenwart der gewünschten Menge an Wasser intensiv vermischt. Hierbei setzt spontan eine exotherme Reaktion unter Bildung des 2-Halogenpyridincarbonsäureamids ein. In der Regel wird man die Umsetzung oberhalb Raumtemperatur, vorzugsweise jedoch oberhalb 40 °C und insbesondere oberhalb 50 °C durchführen. Vorzugsweise geht man so vor, daß man die Lösungen der Edukte I und II in dem organischen Lösungsmittel in Gegenwart von Wasser bei einer Temperatur oberhalb 40 °C und insbesondere oberhalb 50°C, z.B. 60 bis 65 °C unter quasi-adiabatischen Bedingungen miteinander vermischt, so daß es zu einer Erwärmung des Reaktorinhalts kommt. Unter quasi-adiabatischen Bedingungen versteht man hierbei solche Reaktionsbedingungen, bei denen die Hauptmenge der bei der Amidbildung freiwerdenden Enthalpie nicht direkt durch Kühlungsvorrichtungen abgeführt wird sondern eine Erwärmung des Reaktorinhaltes bewirkt. Während der Umsetzung sorgt man üblicherweise für eine kräftige Durchmischung, z.B. durch intensives Rühren und/oder durch Umpumpen des Reaktorinhalts. Insbesondere geht man so vor, daß man die gewünschte Menge an Wasser sowie die Lösung des aromatischen Monoamins I in dem gewünschten organischen Lösungsmittel im Reaktionsgefäß vorlegt, den Reaktorinhalt auf die gewünschte Temperatur erwärmt und anschließend die Lösung des 2-Halogenpyridincarbonsäurechlorids in dem organischen Lösungsmittel unter Durchmischen dem Reaktor zuführt. Die Dauer dieser Zufuhr kann wenige Minuten bis mehrere Stunden betragen. Bei der bevorzugten quasi-adiabatischen Fahrweise gibt man die Lösung des 2-Halogenpyridincarbonsäurechlorids II vorzugsweise möglichst rasch, z.B. innerhalb 1 bis 30 min, insbesondere 1 bis 15 min, zu. Die Konzentration des 2-Halogenpyridincarbonsäurechlorids II in dem organischen Lösungsmittel liegt in der Regel im Bereich von 20 Gewichtsteilen bis 200 Gewichtsteilen je 100 Gew.-Teile organisches Lösungsmittel.

Nach Beendigung der Zugabe läßt man die Bestandteile in der Regel noch eine Zeit lang, vorzugsweise nicht mehr als 1 h nachreagieren, bevor man die Aufarbeitung beginnt. Vorzugsweise erfolgt diese Nachreaktion unter Durchmischen des Reaktorinhaltes.

Die Aufarbeitung erfolgt in an sich üblicher Weise wässrig extraktiv. Hierzu trennt man in der Regel zunächst die wässrige Phase des gegebenenfalls noch heißen Reaktionsgemisches ab. Anschließend wird die organische Phase, gegebenenfalls nach Verdünnen mit weiterem organischen Lösungsmittel, durch zugabe einer wässrigen Lösung einer anorganischen Base neutralisiert. Als Basen kommen beispielsweise Alkalihydroxide wie Natriumhydroxid oder Kaliumhydroxid, Alkalicarbonate und -hydrogencarbonate und insbesondere Natriumcarbonat in Betracht. Die Neutralisation kann in einem oder in mehreren Schritten erfolgen. Vorzugsweise stellt man bei der Neutralisation einen pH-Wert im Bereich von 6 bis 10 und insbesondere im Bereich von 7 bis 9 ein. Vorzugsweise erfolgt der Neutralisationsvorgang ebenfalls bei Temperaturen oberhalb 40 °C, insbesondere oberhalb 60°C, und besonders oberhalb 85 °C, z.B. im Bereich von 60 bis 100 °C oder im Bereich von 85 bis 100 °C. Vorzugsweise gibt man hierbei eine heiße wässrige Basen-Lösung zu der heißen organischen Phase. Die Neutralisation kann in einem oder in mehreren Schritten erfolgen, wobei man jeweils im Anschluß an einen Schritt die wässrige Phase von der organischen Phase abtrennt.

In der Regel wird man für die Gewinnung weiterer Wirkstoffmengen die erste wässrige Phase oder die vereinigten wässrigen Phasen mit dem organischen Lösungsmittel reextrahieren und die dabei erhaltene organische Lösung gegebenenfalls in der oben beschriebenen Weise neutralisieren. Vorzugsweise wird das so gewonnene Re-Extrakt in die Reaktion, z.B. einem nächsten Ansatz zurückgeführt. Selbstverständlich kann man den Wirkstoff auch aus dem Re-Extrakt isolieren.

Die Isolierung des so hergestellten 2-Halogenpyridincarbonsäureamids aus der organischen Phase erfolgt in einer an sich üblichen Weise, z.B. durch Einengen und/oder Abkühlen der organischen Lösungen und Kristallisation. Die Kristallisation kann beispielsweise in Gegenwart von Impfkristallen durchgeführt werden.

Zur Herstellung des 2-Halogenpyridincarbonsäureamids setzt man die Edukte I und II in nahezu stöchometrischer Menge ein, d.h. das Molverhältnis von 2-Halogennicotinsäurechlorid II und aromatischem Amin I liegt im Bereich von 0,9:1 bis 1:1,1. Bevorzugt setzt man jedoch das 2-Halogennicotinsäurechlorid II in wenigstens equimolarer Menge oder in einem geringen Überschuß von bis zu 10 Mol-%, vorzugsweise bis zu 5 Mol-% bezogen auf I ein.

Erfindungsgemäß lassen sich mit dem Verfahren alle primären aromatischen Monoamine II umsetzen, die in der ortho-Position zur Aminogruppe einen von Wasserstoff verschiedenen Substituenten aufweisen. Hierbei kommen grundsätzlich solche Substituenten in Betracht, die unter angegebenen Reaktionsbedingungen inert sind, d.h. mit der Säurechlorid-Funktion des 2-Halogenpyridincarbonsäurechlorids II keine Konkurrenzreaktion eingehen. Beispiele für derartige Substituenten sind Halogen, Nitro, Cyano, Alkyl, Halogenalkyl, Cycloalkyl, Alkoxy, Alkoxyalkyl, Alkylthio, Alkylsulfonyl, Aryl, Heteroaryl, Arylalkyl und Heteroarylalkyl. Die Aryl- und Heteroarylgruppen der vier letztgenannten Reste können dabei ihrerseits 1, 2 oder 3 der vorgenannten Gruppen als Substituenten aufweisen, z.B. Halogen, Nitro, Cyano, Alkyl, Halogenalkyl, Alkoxy, Alkoxyalkyl, Alkylthio, Alkylsulfonyl, Aryl und/oder Cycloalkyl. Neben dem Substituenten in der ortho-Position zur Aminogruppe kann das aromatische Monoamin auch weitere, z.B. 1 oder 2 weitere Substituenten der vorgenannten Art tragen. Zwei an benachbarte C-Atome des Aromaten gebundenen Substituenten können auch gemeinsam einen carbocyclischen oder heterocyclischen 5- oder 6-gliedrigen Ring bilden, der seinerseits substituiert sein kann, z.B. durch Halogen oder Alkyl.

Die aromatischen Monoamine leiten sich vorzugsweise von Anilin ab. Aber auch Amine polycyclischer Aromate wie Naphthylamine oder Amine von Benzoheterocyclen können als Monoamin I eingesetzt werden. Die aromatischen Monoamine, insbesondere die Anilinverbindungen können selbstverständlich neben dem Substituenten in der ortho-Position noch weitere, z.B. 1 oder 2 Substituenten der vorgenannten Art aufweisen. In einer bevorzugten Ausführungsform der vorliegenden Erfindung verwendet man ein Anilin, das ausschließlich in ortho-Position substituiert ist.

Alkyl steht hierbei für einen linearen oder verzweigten gesättigten Kohlenwasserstoffrest mit vorzugsweise 1 bis 6 und insbesondere 1 bis 4 C-Atomen wie Methyl, Ethyl, n-Propyl, Isopropyl, n-Butyl, 2-Butyl, Isobutyl und tert.-Butyl. Gleiches gilt für die Alkylteile in Alkoxy, Alkythio, Alkoxyalkyl und Alkylsulfonyl.

Halogenalkyl steht hierbei für einen teilweise oder vollständig durch Halogen, insbesondere Fluor, Chlor, Brom oder Iod substituierten, linearen oder verzweigten, gesättigten Kohlenwasserstoffrest mit vorzugsweise 1 bis 4 C-Atomen, z.B. für Chlormethyl, Dichlormethyl, Trichlormethyl, Fluormethyl, Difluormethyl, Chlordifluormethyl, Dichlorfluormethyl, Trifluormethyl, Brommethyl, 2,2,2-Trichlorethyl, 2,2,2-Trifluormethyl, 2-Chlorethyl, Pentafluorethyl, Pentachlorethyl, 3-Chlorpropyl, 3-Brompropyl, etc.

Alkoxyalkyl steht für einen linearen oder verzweigten Alkyl-Rest, der durch eine C₁-C₄-Alkoxygruppe substituiert ist, z.B. für Methoxymethyl, Ethoxymethyl, n- oder i-Propoxymethyl, n-Butoxymethyl, 1-Methoxyethyl, 1-Ethoxyethyl, 2-Methoxyethyl, 2-Ethoxyethyl, etc.

Cycloalkyl steht für mono- oder bicyclische Kohlenwasserstoffreste mit in der Regel 3 bis 10 C-Atomen wie Cyclopropyl, Cyclopentyl, Cyclohexyl, Norbornyl, Dekalinyl oder Adamantyl.

Aryl steht vorzugsweise für Phenyl oder für Naphthyl, die gegebenenfalls durch 1, 2 oder 3 der vorgenannten Substituenten substituiert sein können.

Heteroaryl steht für einen heteroaromatischen Rest, der mono- oder bicyclisch sein kann und der 1 bis 3 Heteroatome, ausgewählt unter O, N und S aufweist, wobei keines der Heteroatome im Wässrigen protonierbar ist. Beispiele für Hetaryl sind insbesondere Thienyl, Furanyl, Benzothienyl, Indolyl und dergleichen. Aryl und Hetaryl können durch einen oder mehrere, z.B. 1, 2 oder 3, der vorgenannten Substituenten substituiert sein.

In einer besonders bevorzugten Ausführungsform der vorliegenden Erfindung wird ein primäres aromatisches Monoamin I eingesetzt, das in der ortho-Position zur Aminogruppe einen Phenyl-Substituenten aufweist, welcher seinerseits substituiert sein kann, z.B. 1, 2 oder 3 der vorgenannten Substituenten aufweist. Insbesondere betrifft die vorliegende Erfindung ein Verfahren, bei dem man als aromatisches Monoamin I eine Anilinverbindung einsetzt, die in der ortho-Position zur Aminogruppe einen gegebenenfalls substituierten Phenylring aufweist, also ein aromatisches Monoamin I von 2-Aminobiphenyl-Typ. Der Phenylring kann in der oben beschriebenen Weise substituiert sein und weist vorzugsweise 1, 2 oder 3 der vorgenannten Substituenten auf. Besonders bevorzugte Substituenten sind ausgewählt unter Halogen, Methyl, Difluormethyl, Trifluormethyl, Methoxy, Methylthio oder Methylsulfonyl. In einer speziellen Ausführungsform der vorliegenden Erfindung setzt man als aromatisches Monoamin I ein 2-(Halogenphenyl)anilin, z.B. 2-(4-Chlorphenyl)anilin oder 2-(4-Fluorphenyl)anilin ein.

Bei den im erfindungsgemäßen Verfahren eingesetzten 2-Halogenpyridincarbonsäurechloriden handelt es sich vorzugsweise um 2-Halogennicotinsäurechloride und insbesondere um 2-Chlornicotinsäurechlorid (=2-Chlor-3-pyridincarbonsäurechlorid).

Das erfindungsgemäße Verfahren liefert 2-Halogenpyridincarbonsäureamide von sterisch gehinderten, primären aromatischen Monoaminen I, die in der ortho-Postition zur Aminogruppe einen von Wasserstoff verschiedenen Substituenten aufweisen, in hohen Ausbeuten, ohne daß der Einsatz einer Base erforderlich ist. Die Ausbeuten bei konventioneller Aufarbeitung überschreiten in der Regel 80 % der theoretischen Ausbeute. Durch Re-Extraktion der wässrigen Phase kann die Ausbeute regelmäßig auf über 90 % gesteigert werden. Überraschenderweise wird unter den Reaktionsbedingungen nur eine sehr geringe Menge an 2-Hydroxynicotinsäureamid gebildet, welches üblicherweise bei saurer Hydrolyse von 2-Halogennicotinsäuramiden entsteht.

Das folgende Beispiel soll die Erfindung verdeutlichen, ohne sie jedoch einzuschränken:

Herstellung von 2-Chlor-[2-(4-Chlorphenyl)phenylaminocarbonyl]pyridin durch Umsetzung von 2-Chlor-3-nicotinsäurechlorid II mit 2-(4-Chlorphenyl)anilin I

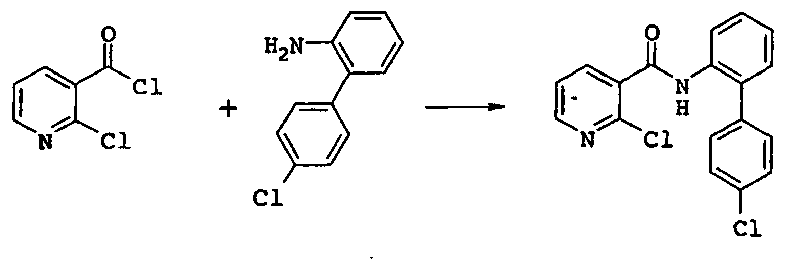

In einem Reaktionsgefäß wurden 800 g Wasser und eine Lösung von 396 g (1,944 mol) 2-Amino-4'-chlorbiphenyl in 311 g Xylol vorgelegt und unter Rühren auf 65°C Innentemperatur erwärmt. Dann gab man hierzu eine auf 65 °C erwärmte Lösung von 349 g (1,984 mol) 2-Chlor-3-nicotinsäurechlorid in 233 g Xylol. Hierbei stieg die Temperatur im Reaktionsgefäß auf etwa 95 °C an. Nach Beendigung der Zugabe behielt man die Temperatur noch weitere 10 min unter Rühren bei, stellte den Rührer ab und ließ die Phasen trennen. Die untere Wasserphase wurde abgelassen und gesammelt. Zu der organischen Phase gab man etwa 360 g heißes Wasser, rührte und gab dann hierzu eine erste Menge einer 20 gew.-%igen, wässrigen Natriumcarbonat-Lösung. Man trennte die wässrige Phase ab und gab erneut zu der organischen Phase 360 g heißes Wasser und weitere 20 Gew.-%ige Natriumcarbonat-Lösung. Nach Abtrennen der wässrigen Phase wurde die heiße organische Phase in ein vorgeheiztes Vorratsgefäß überführt. Man kühlte unter Rühren auf Raumtemperatur. Hierbei kristallisierte die Titelverbindung aus. Nach Abtrennen der Mutterlauge und Trocknen des Kristallisats erhielt man 567 g des Wirkstoffs. Dies entspricht einer Ausbeute von 85 %, bezogen auf das eingesetzte 2-Aminobiphenyl.

Re-Extraktion der wässrigen Phasen mit Xylol lieferte weitere 53 g der Titelverbindung. Die Gesamtausbeute betrug 620 g (93 % der theoretischen Ausbeute).

## Patentansprüche

1. Verfahren zur Herstellung von 2-Halogenpyridincarbonsäureamiden primärer aromatischer Monoamine I, die in der ortho-Position zur Aminogruppe einen von Wasserstoff verschiedenen Substituenten aufweisen, durch Umsetzung von 2-Halogenpyridincarbonsäurechlorid II mit dem aromatischen Monoamin I, **dadurch gekennzeichnet, dass** man die Umsetzung in einer Lösungsmittelmischung aus Wasser und wenigstens einem unter den salzsauren Reaktionsbedingungen mit Wasser nicht mischbaren organischen Lösungsmittel durchführt, das ausgewählt ist unter aromatischen Kohlenwasserstoffen, Halogenkohlenwasserstoffen, acyclischen Ethern mit 4 bis 10 C-Atomen, 3 bis 10 C-Atome aufweisenden Estern aliphatischer oder cycloaliphatischer Alkohole mit aliphatischen Carbonsäuren und Mischungen dieser Lösungsmittel, wobei die Mischung unter den salzsauren Reaktionsbedingungen wenigstens eine organische und wenigstens eine wässrige Phase umfasst, und wobei die Mischung keine oder weniger als 10 mol-%, bezogen auf das 2-Halogenpyridincarbonsäurechlorid II, einer von I und II verschiedenen Base enthält, und die Gesamtmenge von Verbindung I und II 25 Gew.-Teile bis 100 Gew.-Teile, bezogen auf 100 Gew.-Teile organisches Lösungsmittel, beträgt.

2. Verfahren nach Anspruch 1, **dadurch gekennzeichnet, dass** die Wassermenge wenigstens 100 g je mol 2-Halogenpyridincarbonsäurechlorid II beträgt.

3. Verfahren nach einem der vorhergehenden Ansprüche, **dadurch gekennzeichnet, dass** man die Umsetzung bei Temperaturen oberhalb 40°C durchführt.

4. Verfahren nach einem der vorhergehenden Ansprüche, **dadurch gekennzeichnet, dass** man die Umsetzung in Abwesenheit einer von I und II verschiedenen Base durchführt.

5. Verfahren nach einem der vorhergehenden Ansprüche, **dadurch gekennzeichnet, dass** man 2-Halogenpyridincarbonylchlorid II und das aromatische Amin I in einem Molverhältnis von 1:1,1 bis 1:1 umsetzt.

6. Verfahren nach einem der vorhergehenden Ansprüche, **dadurch gekennzeichnet, dass** der Substituent in der ortho-Position der Aminogruppe in I eine Phenylgruppe ist, die gegebenenfalls ihrerseits substituiert ist.

7. Verfahren nach Anspruch 6, **dadurch gekennzeichnet, dass** das aromatische Amin I ausgewählt ist unter 2-(Halogenphenyl) anilinen.

8. Verfahren nach einem der vorhergehenden Ansprüche, **dadurch gekennzeichnet, dass** man als Verbindung II 2-Chlorpyridincarbonsäurechlorid einsetzt.

## Claims

1. A process for preparing 2-halopyridinecarboxamides of primary aromatic monoamines I which have a substituent other than hydrogen in the ortho-position to the amino group by reaction of 2-halopyridinecarbonyl chloride II with the aromatic monoamine I, which comprises carrying out the reaction in a solvent mixture comprising water and at least one organic solvent which is water-immiscible under the hydrochloric acid reaction conditions and is selected from aromatic hydrocarbons, halogenated hydrocarbons, acyclic ethers having from 4 to 10 carbon atoms, C₃-C₁₀ esters of aliphatic or cycloaliphatic alcohols with aliphatic carboxylic acids and mixtures of these solvents, the mixture comprising at least one organic and at least one aqueous phase under the hydrochloric acid reaction conditions, and the mixture comprising none or less than 10 mol%, based on the 2-halopyridinecarbonyl chloride II, of a base other than I or II, and the total quantity of compounds I and II being from 25 parts by weight to 100 parts by weight, based on 100 parts by weight of organic solvent.

2. The process according to claim 1, wherein the water quantity is at least 100 g per mole of 2-halopyridinecarbonyl chloride II.

3. The process according to any of the preceding claims, wherein the reaction is carried out at temperatures above 40°C.

4. The process according to any of the preceding claims, wherein the reaction is carried out in the absence of a base other than I or II.

5. The process according to any of the preceding claims, wherein the 2-halopyridinecarbonyl chloride II and the aromatic amine I are reacted in a molar ratio of from 1:1.1 to 1:1.

6. The process according to any of the preceding claims, wherein the substituent in the ortho-position of the amino group in I is a phenyl group which is itself optionally substituted.

7. A process according to claim 6, wherein the aromatic amine I is selected from 2-(halophenyl)anilines.

8. The process according to any of the preceding claims, wherein compound II is 2-chloropyridinecarbonyl chloride.

## Revendications

1. Procédé de préparation d'amides d'acide 2-halogénopyridinecarboxylique de monoamines aromatiques primaires I, qui présentent, dans la position ortho par rapport au groupe amino, un substituant différent de l'hydrogène, par réaction de chlorure d'acide 2-halogénopyridinecarboxylique II avec la monoamine aromatique I, **caractérisé en ce qu'**on effectue la réaction dans un mélange de solvants à base d'eau et d'au moins un solvant organique non miscible à l'eau dans les conditions réactionnelles chlorhydriques qui est choisi parmi des hydrocarbures aromatiques, des hydrocarbures halogénés, des éthers acycliques comportant 4 à 10 atomes de C, des esters présentant 3 à 10 atomes de C d'alcools aliphatiques ou cycloaliphatiques avec des acides carboxyliques aliphatiques et des mélanges de ces solvants, le mélange comportant dans les conditions réactionnelles chlorhydriques au moins une phase organique et au moins une phase aqueuse, le mélange ne contenant aucune base différente de I et de II ou moins de 10 % molaires, par rapport au chlorure d'acide 2-halogénopyridinecarboxylique II, et la quantité totale des composés I et II étant de 25 parties en poids à 100 parties en poids, par rapport à 100 parties en poids du solvant organique.

2. Procédé suivant la revendication 1, **caractérisé en ce que** la quantité d'eau est d'au moins 100 g par mole de chlorure d'acide 2-halogénopyridinecarboxylique II.

3. Procédé suivant l'une des revendications précédentes, **caractérisé en ce qu'**on effectue la réaction à des températures supérieures à 40°C.

4. Procédé suivant l'une des revendications précédentes, **caractérisé en ce qu'**on effectue la réaction en l'absence d'une base différente de I et II.

5. Procédé suivant l'une des revendications précédentes, **caractérisé en ce qu'**on fait réagir du chlorure de 2-halogénopyridinecarbonyle II et l'amine aromatique I dans un rapport molaire de 1/1,1 à 1/1.

6. Procédé suivant l'une des revendications précédentes, **caractérisé en ce que** le substituant dans la position ortho du groupe amino dans I est un groupe phényle qui, éventuellement, est substitué à son tour.

7. Procédé suivant la revendication 6, **caractérisé en ce que** l'amine aromatique I est choisie parmi des 2-(halogénophényl)-anilines.

8. Procédé suivant l'une des revendications précédentes, **caractérisé en ce que**, comme composé II, on met en oeuvre du chlorure d'acide 2-chloropyridinecarboxylique.
